(19) Europäisches Patentamt
European Patent Office
Office européen des brevets

(11) EP 0 664 999 B1

(12) EUROPÄISCHE PATENTSCHRIFT

(45) Veröffentlichungstag und Bekanntmachung des Hinweises auf die Patenterteilung:
**24.03.1999 Patentblatt 1999/12**

(51) Int. Cl.[6]: **A61K 6/083**, A61K 6/10, A61K 6/00

(21) Anmeldenummer: **95250014.8**

(22) Anmeldetag: **23.01.1995**

(54) **Verwendung einer thermisch härtbaren Zusammensetzung als Dentalmaterial**
Use of heat curable compositions as dental material
Utilisation de compositions thermodurcissables comme matériaux dentaires

(84) Benannte Vertragsstaaten:
**AT CH DE FR GB IT LI SE**

(30) Priorität: **26.01.1994 DE 4402766**

(43) Veröffentlichungstag der Anmeldung:
**02.08.1995 Patentblatt 1995/31**

(73) Patentinhaber: **IVOCLAR AG**
**FL-9494 Schaan (LI)**

(72) Erfinder:
- **Rheinberger, Volker, Dr.**
**FL-9490 Vaduz (LI)**
- **Moszner, Norbert, Prof. Dr.**
**FL-9492 Eschen (LI)**

(74) Vertreter: **UEXKÜLL & STOLBERG**
**Patentanwälte**
**Beselerstrasse 4**
**22607 Hamburg (DE)**

(56) Entgegenhaltungen:
**EP-A- 0 461 586** **DE-A- 4 141 174**
**US-A- 4 882 392**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Xerox (UK) Business Services
2.16.7/3.6

## Beschreibung

Die Erfindung betrifft die Verwendung einer thermisch härtbaren Zusammensetzung als Dentalmaterial oder Bestandteil eines Dentalmaterials sowie daraus geformte Dentalkörper.

Die Anforderungen, die an Zusammensetzungen gestellt werden, welche als Dentalmaterialien oder Bestandteile von diesen eingesetzt werden sollen, sind vielfältig und je nach Art des Dentalmaterials unterschiedlich. Dabei ist eine wesentliche, an alle Dentalmaterialien gestellte Forderung, daß diese unter den in der Mundhöhle vorherrschenden Bedingungen stabil sind und insbesondere in Folge eines etwaigen Kontakts mit Speichel nicht hydrolysieren. Bei härtbaren Dentalmaterialen, wie z.B. Füllungsmaterialien und Polymermaterialien zur Herstellung von künstlichen Zähnen, wird besonderer Wert darauf gelegt, daß beim Härtungsvorgang nur ein geringer Volumenschrumpf auftritt. Ist dieser hingegen zu groß, so sind Paßungenauigkeiten des ausgehärteten Materials unweigerlich die Folge. Weiter sollten härtbare Dentalmaterialen, die in der Mundhöhle in direktem Kontakt mit Nahrungsmitteln treten, sich durch eine hohe Härte auszeichnen. Schließlich ist es überdies besonders wünschenswert, daß der Aushärtungsvorgang auf einfache Weise ohne Zuhilfenahme von aufwendigen Gerätschaften durchgeführt werden kann.

Polymere, die auf einfache Weise, nämlich thermisch bei Raumtemperatur, aushärten, sind bekannt. Zu ihrer Herstellung wird von Vinylmonomeren ausgegangen. Diese werden durch radikalische Polymerisation mit Redoxinitiatorsystemen, wie z.B. Peroxid/Amin-Kombinationen oder mit Boralkylen durch kationische Polymerisation mit Säure-Katalysatoren oder durch anionische Polymerisation unter Verwendung von Gruppentransferkatalysatoren zu den gewünschten Polymeren umgesetzt (vgl. Houben-Weyl: Methoden der anorganischen Chemie, Band E20, Teil 1, Seiten 15, 94 und 153, Thieme-Verlag, Stuttgart 1986). Die radikalische oder kationische Polymerisation kann dabei ebenfalls mittels Photoinitiatoren eingeleitet werden (vgl. H.J. Timpe, H. Baumann, Photopolymere: Prinzipien und Anwendungen, Deutscher Verlag für Grundstoffindustrie, Leipzig 1988, Seiten 49 und 95).

Nachteilig an diesen bekannten Polymeren ist, daß die zu ihrer Aushärtung durchgeführten Polymerisationsverfahren empfindlich gegenüber der Anwesenheit von z.B. Sauerstoff und Wasser sind. So wird die radikalische Polymerisation durch Sauerstoff und die anionische und kationische Polymerisation durch Wasser oder elektrophile bzw. nucleophile Verunreinigungen stark inhibiert. Weiterhin tritt bei Durchführung einer vernetzenden Polymerisation ein starker Volumenschrumpf auf mit der Folge, daß auf ein Substrat aufgebrachte Polymerisate zum Abheben und Abplatzen von der Substratoberfläche neigen.

Weitere Polymermaterialien, die bei Raumtemperatur gehärtet werden können, sind Polyurethane oder Polyharnstoffe, die durch Reaktion von Diolen oder Diaminen mit Di- oder Multiisocyanaten hergestellt werden (vgl. H.-G. Elias, Makromoleküle, Band 2, Seite 225, Hüthig & Wepf Verlag, Basel, Heidelberg, New York 1992). Einschränkend wirkt sich bei diesen Polymersystemen jedoch die hohe Wasserempfindlichkeit der Isocyanatkomponente aus. Außerdem sind für eine hinreichend schnelle Aushärtung toxische Katalysatoren, wie z.B. zinnorganische Verbindungen, erforderlich.

Aus den US-Patenten 4,408,018 und 5,017,649 sind Polymere mit Acetoacetat- oder Acrylat-Gruppen bekannt, die durch Michael-Reaktion mit di- oder polyfunktionellen Acrylaten bzw. Acetoacetaten vernetzt werden können. Diese Polymere sind jedoch nicht als Dentalmaterialien, sondern lediglich als Überzugsmassen vorgesehen.

Gemäß R.J. Clemens, F. Del Rector, J. Coating Techn. 61, 83 (1989) können derartige vernetzte Polymere als Anstrichstoffe eingesetzt werden, die allerdings lediglich eine geringe Hydrolysestabilität zeigten. Als Grund für diesen Nachteil wird angenommen, daß die eingesetzte Amidin-Base in der fertigen Beschichtung die Hydrolyse des vernetzten Films katalysiert.

Schließlich ist aus D.L. Trumbo, Polymer Bull. 26, 265 (1991) bekannt, daß selbst bei Reaktionszeiten von mehr als 24 Stunden verschiedene Bisacetoacetate, wie 1,3- oder 1,4-Bisacetoacetoxymethylbenzol oder 2,2-Dimethyl-1,3-bis(acetoacetyl)-propandiol mit Tripropylenglycoldiacrylat zu Polymeren mit einer mittleren Molmasse von lediglich 2.500 bis höchstens 15.000 reagieren.

Der Erfindung liegt die Aufgabe zugrunde, die Verwendung von Zusammensetzungen als Dentalmaterial oder Bestandteil von Dentalmaterial zur Verfügung zu stellen, welche bei niedriger Temperatur und innerhalb eines kurzen Zeitraums thermisch härtbar sind, sich durch eine gute Hydrolysestabilität und einen geringen Volumenschrumpf bei Härtung auszeichnen, sowie geformte Dentalkörper zu schaffen, welche sich insbesondere durch Hydrolysestabilität und hohe Härte auszeichnen.

Diese Aufgabe wird durch die Verwendung gemäß den Ansprüchen 1 bis 13 sowie den geformten Dentalkörper gemäß den Ansprüchen 14 und 15 gelöst.

Die erfindungsgemäße Verwendung einer thermisch härtbaren Zusammensetzung als Dentalmaterial oder Bestandteil eines Dentalmaterials ist dadurch gekennzeichnet, daß die Zusammensetzung

(a) eine oder mehrere β-Dicarbonylverbindungen der Formel I als Michael-Donor,

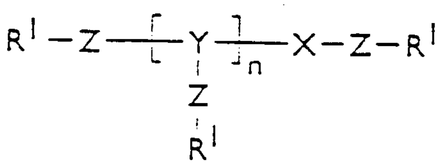

(I)

in der $R^1$ eine β-Dicarbonyl-Funktion der Formel Ia

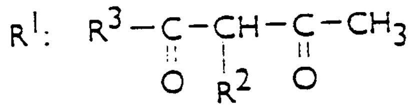

(Ia)

darstellt, $R^2$ Wasserstoff, Alkyl oder Aryl bedeutet, $R^3$ Sauerstoff oder NH ist oder entfällt, Y und X Alkylen-, Phenylen- oder Alkylphenylen-Reste sind, die durch Sauerstoffatome, Schwefelatome oder NH-Gruppen unterbrochen sein können, Z Alkylen oder Phenylen bedeutet und n eine ganze Zahl im Bereich von 0 bis 15 ist, und

(b) einen oder mehrere α,β-ungesättigen Carbonsäureester der Formel II als Michael-Akzeptor enthält,

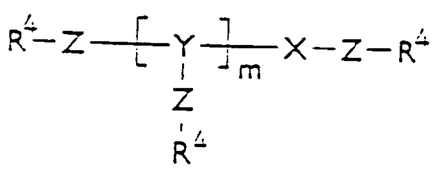

(II)

in der $R^4$ eine Acrylat-Gruppe der Formel IIa

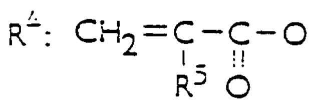

(IIa)

ist, Y, X und Z wie oben definiert sind und $R^5$ Wasserstoff, eine Cyano- oder eine Alkylgruppe ist und m eine ganze Zahl im Bereich von 0 bis 15 ist,

wobei die mittlere Funktionalität der Mischung aus den Michael-Donoren (a) und den Michael-Akzeptoren (b) größer als 2 ist.

Unter Alkyl und Alkylen werden bevorzugt solche Gruppen verstanden, die 1 bis 25, besonders bevorzugt 1 bis 10 und ganz besonders bevorzugt 1 bis 4 Kohlenstoffatome enthalten und ggf. auch einen oder mehrere Substituenten, wie z.B. Halogenatome, Nitrogruppen oder Alkoxyreste tragen. Mit Aryl sind Reste gemeint, die insbesondere 6 bis 14 Kohlenstoffatome aufweisen und wie vorstehend angegeben substituiert sein können.

Die einzelnen Reste $R^1$, $R^2$, $R^3$, $R^4$, $R^5$, X, Y und Z können jeweils gleich oder verschieden sein. So ist es z.B. möglich, daß ein α,β-ungesättigter Carbonsäureester mit unterschiedlichen Acrylatgruppen $R^4$ eingesetzt wird.

Die bevorzugt als Michael-Donor (a) einsetzbaren β-Dicarbonylverbindungen der Formel I sind Acetoacetate und insbesondere solche Acetoacetate, die 3 oder 4 Acetoacetoxy-Gruppen aufweisen, bei denen $R^2$ = Wasserstoff ist. Diese Acetoacetate können aus den entsprechenden Di- oder Polyolen durch Umsetzung mit Diketen (vgl. R.J. Clemens, Chem. Rev. 86, 241 (1986)) bzw. dem Diketen-Aceton-Addukt 2,2,6-Trimethyl-1,3-dioxin-4H-on (vgl. R.J. Clemens, J.A. Hyatt, J. Org. Chem. 50, 2431 (1985)) oder durch Umesterung mit tert.-Butylacetoacetat (vgl. J.C. Gilbert, T.A. Kelly, J. Org. Chem. 53, 449 (1988)) hergestellt werden.

Besonders bevorzugte Michael-Donoren (a) sind Alkandiolbisacetoacetate, insbesondere Ethylenglycol-1,2- oder Hexandiol-1,6-bisacetoacetat, Oxyalkylendiolbisacetoacetate, insbesondere Triethylenglycolbisacetoacetat oder Polyethylenglycol-600-bisacetoacetat, sowie 1,4-Cyclohexandimethanolbisacetoacetat, Glycerintrisacetoacetat und

Pentaerythrittetrakisacetoacetat.

Die als Michael-Akzeptor (b) einsetzbaren $\alpha,\beta$-ungesättigten Carbonsäureester der Formel II sind entweder Handelsprodukte oder durch Veresterung der entsprechenden Di- oder Polyole mit Acrylsäurechlorid oder -anhydrid zugänglich. Bevorzugt werden als Michael-Akzeptor (b) Acrylsäureester eingesetzt, bei denen $R^5$ = Wasserstoff ist, und insbesondere solche, die 3 oder 4 Acrylatgruppen aufweisen. Ganz besonders bevorzugte Michael-Akzeptoren sind Ethylenglycoldiacrylat, Hexandioldiacrylat, Tripropylenglycoldiacrylat, ethoxyliertes Bisphenol-A-diacrylat, Polyethylenglycol-200-diacrylat, Trimethylolpropantriacrylat und Pentaerythrittetraacrylat.

Die Mischung aus den Michael-Donoren und den Michael-Akzeptoren ist derart gewählt, daß deren mittlere Funktionalität größer als zwei ist. Für den Fall einer Mischung aus einer Michael-Donor- und einer Michael-Akzeptor-Verbindung ergibt sich die mittlere Funktionalität $F_m$ aus nachstehender Gleichung:

$$F_m = [(a_{MDG} \cdot n_{MD}) + (a_{AG} \cdot n_{MA})]/(n_{MD} + n_{MA})$$

$a_{MDG}$ = Anzahl der abstrahierbaren H-Atome in einem Michael-Donor-Molekül;
$n_{MD}$ = Molzahl Michael-Donor;
$A_{AG}$ = Anzahl der Acrylatgruppen in einem Michael-Akzeptor-Molekül;
$n_{MA}$ = Molzahl Michael-Akzeptor.

Mit dem Begriff "abstrahierbare H-Atome" sind solche Wasserstoffatome gemeint, die direkt an das Kohlenstoffatom der Gruppe $R^1$ gebunden sind, welches sich zwischen den beiden Carbonylgruppen befindet.

Vorzugsweise ist die Mischung aus Michael-Donoren und -Akzeptoren so beschaffen, daß $n + m \neq 0$ ist. Das bedeutet, daß zumindest auch ein gewisser Anteil an entweder $\beta$-Dicarbonylverbindungen mit mehr als zwei $\beta$-Dicarbonylfunktionen $R^1$ oder ein gewisser Anteil an $\alpha,\beta$-ungesättigten Carbonsäureestern mit mehr als zwei Acrylat-Gruppen $R^4$ vorhanden ist.

Die Härtung der erfindungsgemäß eingesetzten Zusammensetzungen erfolgt vorzugsweise in Gegenwart einer Katalysatorbase (c). Dabei können die Zusammensetzungen die Katalysatorbase auch in einer Weise enthalten, die deren vorzeitigen Kontakt mit sowohl den Michael-Donoren als auch den Michael-Akzeptoren verhindert. Es ist z.B. möglich, daß die Katalysatorbase in einer Verpackung getrennt von Michael-Donoren als auch Michael-Akzeptoren vorliegt. Die Katalysatorbase bewirkt die Katalyse der Michael-Reaktion zwischen dem Michael-Donor und dem Michael-Akzeptor. Aufgrund der stattfindenden Michael-Reaktion erfolgt eine Härtung der erfindungsgemäß eingesetzten Zusammensetzung, die selbst bei niedrigen Temperaturen im Bereich von vorzugsweise 15 bis 80°C und besonders bevorzugt 20 bis 50°C innerhalb kurzer Zeit zu festen Polymerisaten führt. Dieses Ergebnis wird überraschenderweise auch dann erzielt, wenn in einer bevorzugten Ausführungsform die Härtung der Zusammensetzung in Abwesenheit von Lösungsmitteln erfolgt, die aufgrund möglicher gesundheitsschädigender Wirkung nachteilig sind.

Als Katalysatorbase (c) werden vorzugsweise Alkalimetallalkoxide, Tetraalkylhydroxide, z.B. Tetraalkylammoniumhydroxid, bicyclische Amidine und Guanidine eingesetzt. Besonders bevorzugte Katalysatoren sind in den Beispielen angegeben.

Die Mischung aus den $\beta$-Dicarbonylverbindungen (a) und den $\alpha,\beta$-ungesättigten Carbonsäureestern (b) ist so gewählt, daß das Molverhältnis von (a) / (b) vorzugsweise 0,01 bis 20 und besonders bevorzugt 0,1 bis 8,0 beträgt.

In einer weiteren bevorzugten Ausführungsform ist die Zusammensetzung so beschaffen, daß bezogen auf den Michael-Donor (a) ein stöchiometrischer Überschuß an Michael-Akzeptor (b) eingesetzt wird. Die Härtung solcher Zusammensetzungen führt zu Polymermaterialien, die nach Abschluß der Michael-Reaktion noch unumgesetzt Acrylatgruppen $R^5$ enthalten, welche in einer 2. Stufe radikalisch polymerisierbar sind. Derartige Materialien werden vorzugsweise als reaktive Füllstoffe von Dentalmaterialien oder als Polymermatrix von Füllungsmaterialien oder Dentalzementen eingesetzt.

In einer weiteren bevorzugten Ausführungsform kann die erfindungsgemäß eingesetzte Zusammensetzung auch ein Modifizierungsmittel (d) enthalten, welches mindestens eine radikalisch polymerisierbare Gruppe aufweist, deren Reaktivität gegenüber dem Michael-Donor (a) geringer ist als die der Acrylat-Gruppen $R^4$ des Michael-Akzeptors (b). Ein Gehalt an Modifizierungsmittel führt ebenfalls zu Zusammensetzungen, die in zwei Stufen ausgehärtet werden können. So führt die Reaktion der Michael-Akzeptoren mit den Michael-Donoren in Gegenwart der Katalysatorbase zu einer deutlichen Viskositätserhöhung der Mischung oder zur Bildung eines mehr oder weniger festen Gels. In einer zweiten Stufe kann dann durch radikalische Polymerisation die Aushärtung zu einem festen Material durchgeführt werden. Für derartige zweistufig aushärtende Zusammensetzungen sind Mischungen aus Tris- oder Tetrakisacetoacetaten zusammen mit Tri- oder Tetraacrylaten geeignet, denen Mono- oder Multimethacrylate als Modifizierungsmittel (d)

zugesetzt werden. Letztere zeigen verglichen mit Tri- oder Tetraacrylaten eine deutlich geringere Reaktivität in einer Michael-Reaktion mit CH-aciden Verbindungen, wie Acetoacetaten, β-Diketonen oder Malonestern. Sie sind jedoch auf der anderen Seite sehr gut radikalisch polymerisierbar. Zweistufig aushärtbare Materialien bieten z.B. den Vorteil, daß eine Formgebung des Materials oder die Entfernung von Überschüssen nach der ersten Härtungsstufe aufgrund der weicheren Eigenschaften einfacher realisiert werden kann.

Bei besonders bevorzugten zweistufig aushärtenden Zusammensetzungen werden als Michael-Donoren (a) Glycerintrisacetoacetat oder Pentaerythrittetrakisacetoacetat, als Michael-Akzeptoren (b) Trimethylolpropantriacrylat oder Pentaerythritoltetraacrylat und als Modifizierungsmittel (d) Methylmethacrylat, Butylmethacrylat, 2-Hydroxyethylmethacrylat (HEMA), 3-Methacryloxypropyl-trimethoxysilan, Triethylenglycoldimethacrylat, Bisphenol-A-glycidylmethacrylat, das Urethandimethacrylat aus 2,2,4-Trimethylhexamethylendiisocyanat und HEMA, Trimethylolpropantrimethacrylat oder Pentaerythrittetramethacrylat verwendet. Bei Einsatz von Dimethacrylaten oder Multimethacrylaten bilden sich sogenannte gegenseitig durchdringende Netzwerke (IPN), die den ausgehärteten Zusammensetzungen eine ausgezeichnete Härte und Festigkeit verleihen. Schließlich können als Modifizierungsmittel auch bevorzugt Methacrylate mit Acetoacetoxy-Gruppen, wie insbesondere 2-Acetoacetoxyethylmethacrylat (AAEMA), verwendet werden. Durch Einsatz solcher Modifizierungsmittel sind Zusammensetzungen mit hoher Härte erhältlich.

Sofern eine Funktionalisierung der ausgehärteten Zusammensetzungen gewünscht ist, können diesen vor der Härtungsreaktion monomere Acetoacetate oder Acrylate mit nichtaciden funktionellen Gruppen, wie z.B. Amino-, Hydroxy-, Nitro- oder Halogengruppen, oder Acrylnitril- oder Vinylketon-Derivate hinzugefügt werden. Gehärtete Zusammensetzungen mit Gehalt an freien OH- oder Epoxy-Gruppen können z.B. durch Umsetzung einer Mischung aus Pentaerythrittetrakisacetoacetat und Trimethylolpropantriacrylat mit 2-Hydroxyethylacrylat oder Glycidylacrylat erhalten werden.

Die erfindungsgemäß eingesetzten Zusammensetzungen können mit organischen oder anorganischen partikulären Füllstoffen oder Fasern verstärkt werden, wodurch Kompositmaterialien entstehen. Als Füllstoffe eignen sich insbesondere gefällte oder pyrogene Kieselsäuren, Calciumcarbonat und Calicumhydroxid, Glasfüller oder röntgenopake Stoffe, wie z.B. Ytterbiumfluorid, Bariumsulfid und Bariumhydroxid. Als Fasern sind Kurz- oder Langglasfasern sowie Cellulose- oder Polyamidfasern bevorzugt. Eine verbesserte Haftung oder Einbindung der Füllstoffe in die Michael-Polymermatrix kann durch Silanisierung mit Acryloxyalkylsilanen, wie z.B. dem käuflichen 3-Acryloxypropyl-trimethoxysilan erreicht werden. Ferner können die Zusammensetzungen üblich Hilfs- und Zusatzstoffe, wie z.B. Farbstoffe, Pigmente, Thixotropiehilfsmittel, Stabilisatoren, Aromastoffe oder mikrobiocide Wirkstoffe enthalten.

Die erfindungsgemäß verwendeten Zusammensetzungen zeichnen sich durch eine Kombination von Eigenschaften aus, die für Dentalmaterialien eine besondere Bedeutung besitzen. So zeigen sie eine nur geringe Empfindlichkeit gegenüber Wasser, die sich in einer nur geringen Änderung der mechanischen Eigenschaften bei mehrtägiger Lagerung in Wasser manifestiert. Auch können die Zusammensetzungen durch die Wahl der eingesetzten Michael-Donoren und Michael-Akzeptoren so variiert werden, daß gehärtete Materialien mit mechanischen Eigenschaften von hart und spröde bis weich und elastisch erhalten werden.

Wenn die Zusammensetzungen als ungeformte Massen eingesetzt werden, so sind bevorzugte Dentalmaterialien Füllungskomposite, Dentalkleber, Befestigungszemente oder Abformmaterialien. Demgegenüber können die Zusammensetzungen auch geformte Dentalkörper ergeben, die aus der gehärteten Zusammensetzung gebildet sind. Diese Dentalkörper werden vorzugsweise zum Ersetzen oder Restaurieren von Zähnen, d.h. von natürlichen oder künstlichen Zähnen, eingesetzt. Sie liegen bevorzugt in Form eines künstlichen Zahns, eines Inlays, eines Onlays, einer Krone, einer Prothese oder eines Teils einer Prothese vor.

Bei Verwendung der Zusammensetzungen zum Verbinden von Substraten mit natürlichen oder künstlichen Zähnen oder mit Prothesen werden die Zusammensetzungen auf den ausgewählten Bereich des Zahnes oder der Prothese aufgebracht, das Substrat mit der aufgebrachten Zusammensetzung in Kontakt gebracht und die Zusammensetzungen gehärtet.

Wenn die Zusammensetzungen als Abformmaterialien eingesetzt werden sollen, so werden sie auf den abzuformenden Bereich eines natürlichen oder künstlichen Zahnes oder eines Gebisses aufgebracht und gehärtet.

Bei Einsatz der Zusammensetzung zum Restaurieren oder Ersetzen von natürlichen oder künstlichen Zähnen wird die Zusammensetzung (a) in gewünschter Weise verformt, (b) in dem ausgewählten Bereich des Zahnes oder des Gebisses angeordnet und (c) gehärtet, wobei der Verfahrensschritt (c) zeitlich auch zwischen (a) und (b) liegen kann.

Im folgenden wird die Erfindung anhand von Beispielen näher erläutert.

## Beispiele

In den nachstehenden Beispielen werden die folgenden Verbindungen eingesetzt.

Michael-Donoren (a): Hexanediol-1,6-bisacetoacetat (HDDAA),
Polyethylenglycol-600-bisacetoacetat (PEG-600-DAA),

| | |
|---|---|
| | Glycerintrisacetoacetat (GTAA), |
| | Pentaerythrittetrakisacetoacetat (PETAA); |
| Michael-Akzeptoren (b): | Hexandiol-1,6-diacrylat (HDDA), |
| | Polyethylenglycol-400-diacrylat (PEG-400-DA), |
| | Trimethylolpropantriacrylat (TMPTA), |
| | Pentaerythrittetraacrylat (PETA); |
| Modifizierungsmittel: | Methylmethacrylat (MMA), |
| | Triethylenglycoldimethacrylat (TEGDMA), |
| | 2-Acetoacetoxyethylmethacrylat (AAEMA); |
| Katalysatorbasen: | Diazabicyclo[4.3.0]nonen (DBN), |
| | Diazabicyclo[5.4.0]undecen (DBU,) |
| | Tetramethylguanidin (TMG); |
| Photoinitiatoren: | Mischung aus Campferchinon (CC) und |
| | N-(2-Cyanomethyl)-N-methylanilin (CEMA); |
| Füllstoffe: | Bariumglas (BaG), |
| | Ytterbiumfluorid ($YbF_3$), |
| | hochdisperse Kieselsäure (Ox 50 oder Aerosil 200); |
| | $SiO_2$-$ZrO_2$-Mischoxid (Sphärosil). |

Beispiel 1

Herstellung von festen Dentalmaterialien

Es wurden Zusammensetzungen aus verschiedenen Acrylaten mit Acetoacetaten hergestellt und mit 2,0 mol-% DBN - bezogen auf die eingesetzte Acetoacetat-Menge - versetzt. Die Verarbeitungsbreite der Mischungen und ausgewählte Eigenschaften der ausgehärteten Zusammensetzungen sind in Tabelle 1 aufgeführt:

Tabelle 1

| Acrylat/Acetoacetat | HDDA/PETAA | TMPTA/PETAA | PETA/GTAA |
|---|---|---|---|
| Verarbeitungsbreite (Min.) | 7 | 2 | 1,5 |
| Shore-D-Härte | 52±1 | 64±3 | 83±2 |
| Biegefestigkeit (MPa) | nd | 62,1 | 73,2 |
| E-Modul (MPa) | nd | 1960 | 2200 |
| Wasseraufnahme (7d, %) | nd | 5,5 | 5,8 |
| Wasserlöslichkeit (%) | nd | 1,1 | 0,6 |
| nd = nicht bestimmt | | | |

Die oben angegebenen Parameter wurden nach der Iso-Norm 4049 bestimmt.

Die ungehärteten Zusammensetzungen können als Dentaladhäsive oder als Matrixpolymere von Dentaladhäsiven und Befestigungszementen eingesetzt werden.

Beispiel 2

Herstellung von weichen Abformmaterialien

Es wurden Kombinationen aus verschiedenen funktionalisierten Acrylaten mit den Bisacetoacetaten PEG-600-DAA bzw. HDDAA hergestellt und bei Raumtemperatur umgesetzt. Dabei war das Molverhältnis der Acrylat- und Acetoacetat-Gruppen 1 : 1, und es wurden 2,0 mol-% DBN als Katalysatorbase verwendet. Die Verarbeitungsbreite (VB) der Mischungen und die Shore-A-Härte der ausgehärteten Materialien sind in Tabelle 2 zusammengestellt:

Tabelle 2

| Acrylat | PEG-600-DAA VB (Min.)/Shore-A-Härte | HDDAA VB(Min.)/Shore-A-Härte |
|---|---|---|
| PEG-400-DA | 8/31±1 | 3/38±1 |
| HDDA | 6/30±1 | 4/39±1 |
| TMPTA | 2/52±3 | 1/66±1 |
| PETA | 0,5/67±3 | 0,8/74±2 |

Es zeigt sich, daß die Shore-A-Härte der Materialien durch die Funktionalität der Acrylat-Komponente reguliert werden kann. Dabei kommt es mit zunehmender Funktionalität zu einem Anstieg der Härte.

Darüber hinaus können die weichen Materialien durch Füllung weiter verfestigt werden, was am Beispiel von zwei PEG-600-DAA-Materialien (A und B) erkennbar ist.

Tabelle 3

| | A | B |
|---|---|---|
| Zusammensetzung: | | |
| PEG-600-DAA | 62,5 | 64,2 |
| PETA | - | 22,5 |
| TMPTA | 24,4 | - |
| DBN | 0,6 | 0,4 |
| Aerosil 200 | 12,5 | 12,5 |
| Shore-A-Härte | 79±2 | 82±1 |

Beispiel 3

Komposite als Füllungsmaterialien

Es wurden zwei Füllungsmaterialien mit einem Füllungsgrad von 60 (Material 1) bzw. 76,7 Gew.-% (Material 2) auf der Basis einer PETAA/PETA-Mischung und mit folgender Zusammensetzung hergestellt:

Tabelle 4

| Komponente | Material 1 | Material 2 |
|---|---|---|
| PETAA | 22,6 | 14,4 |
| PETA | 16,8 | 8,5 |
| OX 50 | 41,3 | - |
| $YbF_3$ | 18,7 | 17,1 |
| BaG | - | 43,1 |
| Sphärosil | - | 16,5 |
| DBN | 0,6 | 0,4 |

Nach Aushärtung der Materialien bei Raumtemperatur (30 min) und einer 3-stündigen Lagerung bei 50°C wurden folgende Eigenschaften bestimmt:

Tabelle 5

| Eigenschaft | Material 1 | Material 2 |
|---|---|---|
| Volumenschrumpf (%) | 2,2 | 1,8 |
| Biegefestigkeit (MPa) | 24,1 | 43,8 |
| Biege-E-Modul (MPa) | 2300 | 7100 |
| Wasseraufnahme (%) | nd | 2,7 |
| nd = nicht bestimmt. | | |

Das Material 2 kann in vorteilhafter Weise auch zur Bildung von geformten Dentalkörpern eingesetzt werden.

<u>Beispiel 4</u>

<u>Zweistufig härtende Zusammensetzungen für Füllungsmaterialien und Dentalzemente</u>

a) Zusammensetzung auf der Basis einer Acrylat-Acetoacetat/ Methacrylat-Mischung

Es wurde eine Mischung aus den folgenden Komponenten gebildet:

28,4 Gew.-% PETAA,
21,2 Gew.-% PETA,
49,5 Gew.-% TEGDMA,
0,1 Gew.-% CC,
0,2 Gew.-% CEMA und
0,6 Gew.-% DBN.

Dabei wurde das PETA am Schluß zugegeben. Mit der Mischung wurden verschiedene Probekörper hergestellt, wobei die Verarbeitungsbreite der Mischung 10 Minuten betrug. Die bei Raumtemperatur durchgeführte Michael-Reaktion ergab schließlich ein Material mit einer Shore-A-Härte von 85±4. Durch weitere Lichtpolymerisation (3 Minuten, Spectramat - handelsübliche Blaulichtlampe, Hersteller: IVOCLAR AG, Liechtenstein) wurde das Material deutlich härter, was in einer Shore-D-Härte von 75±4, einer Biegefestigkeit von 57,4 MPa und einem Biege-E-Modul von 1420 MPa zum Ausdruck kommt.

b) Zusammensetzung auf der Basis einer Acrylat-Acetoacetat-Mischung mit einem Acrylat-Überschuß:

Bei Zusammensetzungen auf der Basis von PETAA-Mischungen mit verschiedenem PETA-Gehalt wurde die Shore-Härte nach der 1. Härtungsstufe (Michael-Reaktion) und nach der 2. Härtungsstufe (3 Minuten Lichtpolymerisation im Spectramat) untersucht:

Tabelle 6

| Zusammensetzung (Gew.-%) | Mol Acetoacetat/Mol Acrylat | | | |
|---|---|---|---|---|
| | 1:5 | 1:6 | 1:8 | 1:10 |
| PETAA | 20,9 | 18,1 | 14,2 | 11,7 |
| PETA | 77,9 | 80,7 | 84,7 | 87,3 |
| DBN | 0,4 | 0,4 | 0,3 | 0,2 |
| CEMA | 0,5 | 0,5 | 0,5 | 0,5 |
| CC | 0,3 | 0,3 | 0,3 | 0,3 |
| 1. Stufe: Shore-Härte | 49 D | 90 A | 73 A | 63 A |

Tabelle 6 (fortgesetzt)

| Zusammensetzung (Gew.-%) | Mol Acetoacetat/Mol Acrylat | | | |
|---|---|---|---|---|
| | 1:5 | 1:6 | 1:8 | 1:10 |
| 2. Stufe: Shore-Härte | 72 D | 83 D | 83 D | 84 D |

Es ist erkennbar, daß die Härte nach der 1. Stufe vom Acrylatüberschuß abhängt und daß in allen Beispielen die Festigkeit der Materialien durch die Lichtpolymerisation deutlich erhöht werden kann.

Mit gefüllten und ungefüllten Materialien auf der Basis von PETAA/PETA-Mischungen im Molverhältnis 1:5 wurden Biegeprüfungen durchgeführt. Die in Tabelle 7 dargestellten Ergebnisse belegen, daß sich die mechanischen Eigenschaften nach Wasserlagerung nur wenig verschlechtern. Demgemäß sind Zusammensetzungen, die einen Überschuß an Michael-Akzeptor enthalten, bevorzugt, wenn eine sehr geringe Wasserempfindlichkeit gefordert ist.

Tabelle 7

| Eigenschaft | Ungefülltes Material | Kompositmaterial[a)] |
|---|---|---|
| Biegefestigkeit (MPa) | 64,2 | 70,5 |
| nach 24 h Wasserlagerung | 56,3 | 59,1 |
| Biege-E-Modul (MPa) | 1800 | 5900 |
| nach 24 h Wasserlagerung | 1900 | 5900 |

[a)] Zusammensetzung (Gew.-%): PETAA: 8,4; PETA: 31,2; DBN: 0,2; Ox 50: 41,2; $YbF_3$: 18,7; CC: 0,1; CEMA: 0,2.

Das obige Kompositmaterial kann in vorteilhafter Weise auch zur Herstellung von Dentalkörpern eingesetzt werden.

Wenn den erfindungsgemäßen Zusammensetzungen solche Modifizierungsmittel zugesetzt werden, die neben den radikalisch polymerisierbaren Gruppen auch kovalent in das Polymernetzwerk einbindbare Gruppen aufweisen, so ergeben sich besonders bevorzugte Zusammensetzungen. Dies zeigt das Beispiel 5.

<u>Beispiel 5</u>

<u>Herstellung von funktionalisierten Dentalmaterialien oder Dentalfüllstoffen</u>

a) Zu einer Mischung aus 0,1 mol AAEMA, 0,1 mol GTAA, 0,2 Mol-% DBU (bezogen auf GTAA) und 0,3 Gew.-% CEMA (bezogen auf die Gesamtmasse) werden 0,1 mol PETA gegeben. Nach 25 Min. bei Raumtemperatur und weiterer 3-stündiger Lagerung bei 50°C wird ein Material mit einer Shore-A-Härte von 87 erhalten. Nach Belichtung im Spectramat (3 Minuten) erhöht sich die Härte auf 74 Shore-D.

b) Zu einer Mischung aus 0,1 mol Acetessigsäuremethylester, 0,1 mol GTAA, 0,2 mol-% DBU (bezogen auf GTAA) und 0,3 Gew.-% CC sowie 0,5 Gew.-% CEMA (bezogen auf die Gesamtmasse) werden 0,1 mol PETA gegeben. Nach 25 Min. bei Raumtemperatur und weiterer 3-stündiger Lagerung bei 50°C wird ein Material mit einer Shore-A-Härte von 82 erhalten. Nach Belichtung im Spectramat (3 Minuten) erhöht sich die Härte kaum (84 Shore-A).

c) Zu einer Mischung aus 5 mmol PETAA, 0,2 mol-% DBU (bezogen auf PETAA) und 0,3 Gew.-% CC sowie 0,5 Gew.-% CEMA (bezogen auf die Gesamtmasse) werden 5 mmol PETA und 50 Gew.-% MMA (bezogen auf die Gesamtmasse) gegeben. Nach 25 Min. bei Raumtemperatur und weiterer 3-stündiger Lagerung bei 50°C wird ein Material mit einer Shore-A-Härte von 53 erhalten. Nach Belichtung im Spectramat (3 Minuten) erhöht sich die Härte kaum (54 Shore-A).

Der Vergleich des Experiments (a) mit den Experimenten (b) und (c) zeigt, daß durch die Umsetzung von AAEMA mit der GTAA/PETA-Mischung ein funktionalisiertes Michael-Polymernetzwerk gebildet wird, welches polymerisationsfähige Methacrylatgruppen trägt. Im Gegensatz zu dem im Experiment (c) nur physikalisch gebundenen monofunktionellen MMA läßt sich das gebildete polyfunktionelle Michael-Harz-Methacrylat von (a) sehr gut durch Lichtpolymerisation weiter verfestigen. Dabei belegt das Experiment (b), daß die 2. Aushärtungsstufe nicht aufgrund der Polymerisation von nichtumgesetzter Acrylat-Komponente zustande kommt. Analog lassen sich auch mit anderen funktionalisierten Acrylat- oder Acetoacetat-Komponenten funktionalisierte Dentalmaterialien oder Füllstoffe von Dentalmaterialien herstellen.

Die im diesem Beispiel beschriebenen Zusammensetzungen können z.B. als Dentalklebstoffe oder in einer gefüllten Form als Befestigungszemente oder Füllungskomposite eingesetzt werden.

## Patentansprüche

1. Verwendung einer thermisch härtbaren Zusammensetzung als Dentalmaterial oder Bestandteil eines Dentalmaterials, **dadurch gekennzeichnet**, daß die Zusammensetzung

   (a) eine oder mehrere β-Dicarbonylverbindungen der Formel I als Michael-Donor,

   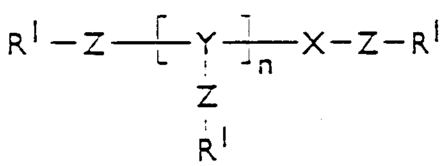

   (I)

   in der $R^1$ eine β-Dicarbonyl-Funktion der Formel Ia

   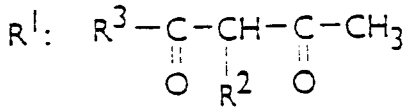

   (Ia)

   darstellt, $R^2$ Wasserstoff, Alkyl oder Aryl bedeutet, $R^3$ Sauerstoff oder NH ist oder entfällt, Y und X Alkylen-, Phenylen- oder Alkylphenylen-Reste sind, die durch Sauerstoffatome, Schwefelatome oder NH-Gruppen unterbrochen sein können, Z Alkylen oder Phenylen bedeutet und n eine ganze Zahl im Bereich von 0 bis 15 ist, und

   (b) einen oder mehrere α,β-ungesättigen Carbonsäureester der Formel II als Michael-Akzeptor enthält,

   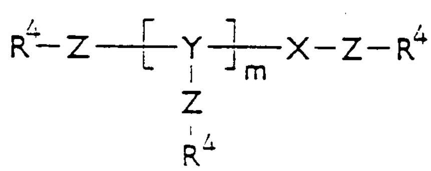

   (II)

   in der $R^4$ eine Acrylat-Gruppe der Formel IIa

   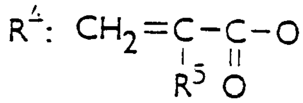

   (IIa)

   ist, Y, X und Z wie oben definiert sind und $R^5$ Wasserstoff, eine Cyano- oder eine Alkylgruppe ist und m eine ganze Zahl im Bereich von 0 bis 15 ist,
   wobei die mittlere Funktionalität der Mischung aus den Michael-Donoren (a) und den Michael-Akzeptoren (b) größer als 2 ist.

2. Verwendung nach Anspruch 1, **dadurch gekennzeichnet**, daß die Zusammensetzung eine Katalysatorbase (c) enthält.

3. Verwendung nach Anspruch 1 oder 2, **dadurch gekennzeichnet**, daß $n + m \neq 0$ ist.

4. Verwendung nach einem der Ansprüche 1 bis 3, **dadurch gekennzeichnet**, daß die Zusammensetzung in Abwesenheit von Lösungmittel gehärtet wird.

5. Verwendung nach einem der Ansprüche 1 bis 4, **dadurch gekennzeichnet**, daß als Michael-Donor (a) Acetoacetate mit drei oder vier Acetoacetoxy-Gruppen eingesetzt werden, bei denen $R^2$ = Wasserstoff ist.

6. Verwendung nach einem der Ansprüche 1 bis 5, **dadurch gekennzeichnet**, daß als Michael-Akzeptor (b) Acrylsäureester mit drei oder vier Acrylatgruppen eingesetzt werden, bei denen $R^5$ = Wasserstoff ist.

7. Verwendung nach einem der Ansprüche 2 bis 6, **dadurch gekennzeichnet**, daß als Katalysatorbase (c) Alkalimetallalkoxide, Tetraalkylammoniumhydroxid, bicyclische Amidine und Guanidine eingesetzt werden.

8. Verwendung nach einem der Ansprüche 1 bis 7, **dadurch gekennzeichnet**, daß bezogen auf den Michael-Donor (a) ein stöchiometrischer Überschuß an Michael-Akzeptor (b) eingesetzt wird.

9. Verwendung nach einem der Ansprüche 1 bis 8, **dadurch gekennzeichnet**, daß die Zusammensetzung ein Modifizierungsmittel (d) enthält, welches mindestens eine radikalisch polymerisierbare Gruppe aufweist, deren Reaktivität gegenüber dem Michael-Donor (a) geringer ist als die der Acrylat-Gruppen $R^4$ des Michael-Akzeptors (b).

10. Verwendung nach einem der Ansprüche 1 bis 9, **dadurch gekennzeichnet**, daß die Zusammensetzung als Füllstoff eines Dentalmaterials eingesetzt wird.

11. Verwendung nach einem der Ansprüche 1 bis 9, **dadurch gekennzeichnet**, daß das Dentalmaterial ein Füllungskomposit, ein Dentalkleber, ein Befestigungszement oder ein Abformmaterial ist.

12. Verwendung nach einem der Ansprüche 1 bis 11, **dadurch gekennzeichnet**, daß die Zusammensetzung in gehärteter Form eingesetzt wird.

13. Verwendung nach Anspruch 12, **dadurch gekennzeichnet**, daß das Dentalmaterial ein künstlicher Zahn, ein Inlay, ein Onlay oder eine Krone ist.

14. Geformter Dentalkörper zum Ersetzen oder Restaurieren von Zähnen, **dadurch gekennzeichnet**, daß der Dentalkörper aus einer gehärteten Zusammensetzung gebildet ist, wie sie gemäß einem der Ansprüche 1 bis 9 verwendet wird.

15. Dentalkörper nach Anspruch 14, **dadurch gekennzeichnet**, daß der Dentalkörper in Form eines künstlichen Zahnes, eines Inlays, eines Onlays, einer Krone, einer Prothese oder eines Teils einer Prothese vorliegt.

## Claims

1. Use of a heat-curable composition as dental material or constituent of a dental material, characterized in that the composition contains

   (a) one or more β-dicarbonyl compounds of the formula I as Michael donor,

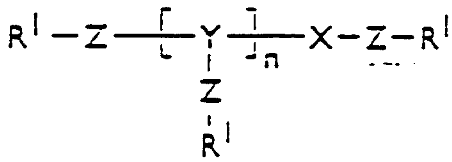

(I)

in which $R^1$ represents a β-dicarbonyl function of formula Ia

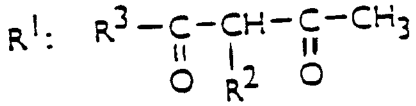

(Ia)

$R^2$ denotes hydrogen, alkyl or aryl, $R^3$ is oxygen or NH or is absent, Y and X are alkylene, phenylene or alkylphenylene radicals which can be interrupted by oxygen atoms, sulphur atoms or NH groups, Z denotes alkylene or phenylene and n is an integer in the range from 0 to 15, and

(b) one or more $\alpha,\beta$-unsaturated carboxylic acid esters of the formula II as Michael acceptor,

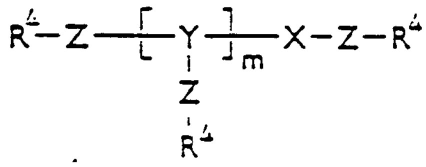

(II)

in which $R^4$ is an acrylate group of the formula IIa

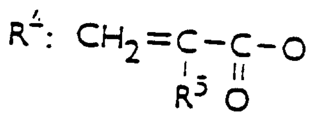

(IIa)

Y, X and Z are as defined above and $R^5$ is hydrogen, a cyano or an alkyl group and m is an integer in the range from 0 to 15,

the average functionality of the mixture comprising the Michael donors (a) and the Michael acceptors (b) being greater than 2.

**2.** Use according to Claim 1, characterized in that the composition contains a catalyst base (c).

**3.** Use according to Claim 1 or 2, characterized in that $n + m \neq 0$.

**4.** Use according to any one of Claims 1 to 3, characterized in that the composition is cured in the absence of solvent.

**5.** Use according to any one of Claims 1 to 4, characterized in that acetoacetates with three or four acetoacetoxy groups in which $R^2$ = hydrogen are used as Michael donor (a).

**6.** Use according to any one of Claims 1 to 5, characterized in that acrylic acid esters with three or four acrylate groups in which $R^5$ = hydrogen are used as Michael acceptor (b).

**7.** Use according to any one of Claims 2 to 6, characterized in that alkali metal alkoxides, tetraalkylammonium hydroxide, bicyclic amidines and guanidines are used as catalyst base (c).

**8.** Use according to any one of Claims 1 to 7, characterized in that, relative to the Michael donor (a), a stoichiometric excess of Michael acceptor (b) is used.

**9.** Use according to any one of Claims 1 to 8, characterized in that the composition contains a modifying agent (d) which has at least one radically polymerizable group whose reactivity vis-à-vis the Michael donor (a) is lower than that of the acrylate groups $R^4$ of the Michael acceptor (b).

**10.** Use according to any one of Claims 1 to 9, characterized in that the composition is used as filler of a dental material.

**11.** Use according to any one of Claims 1 to 9, characterized in that the dental material is a filling composite, a dental adhesive, a fixing cement or an impression material.

**12.** Use according to any one of Claims 1 to 11, characterized in that the composition is used in cured form.

**13.** Use according to Claim 12, characterized in that the dental material is a false tooth, an inlay, an onlay or a crown.

**14.** Moulded dental article for the replacement or restoration of teeth, characterized in that the dental article is formed from a cured composition as is used according to any one of Claims 1 to 9.

**15.** Dental article according to Claim 14, characterized in that the dental article is in the form of a false tooth, an inlay, an onlay, a crown, a prosthesis or a section of a prosthesis.

**Revendications**

**1.** Utilisation d'une composition thermodurcissable comme matériau dentaire ou comme ingrédient d'un matériau dentaire, caractérisée en ce que la composition contient

(a) un ou plusieurs composés β-dicarbonylés de formule I, comme donneurs de Michael,

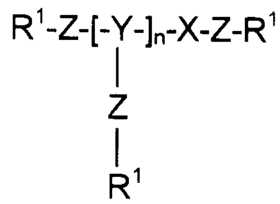

(I)

dans laquelle $R^1$ représente une fonction β-dicarbonyle de formule Ia

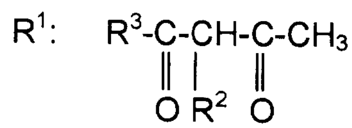

(Ia)

$R^2$ représente un atome d'hydrogène, un groupe alkyle ou un groupe aryle, $R^3$ est un atome d'oxygène ou un groupe NH ou bien ne s'applique pas, Y et X sont des radicaux alkyle, phényle, ou alkylphényle qui peuvent être interrompus par des atomes d'oxygène, des atomes de soufre ou des groupes NH, Z représente des alkyles ou des phényles, et n est un nombre entier dans l'intervalle de 0 à 15, et

(b) un ou plusieurs esters d'un acide carboxylique α,β-insaturés de formule II, comme accepteurs de Michael,

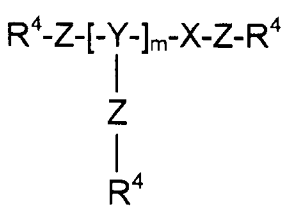

(II)

dans laquelle $R^4$ est un groupe acrylate de formule IIa

$R^4$: 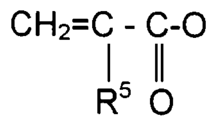 (IIa)

Y, X et Z sont définis comme ci-dessus, et $R^5$ est un atome d'hydrogène, un groupe cyano ou alkyle, et m est un nombre entier dans l'intervalle de 0 à 15,
la fonctionnalité moyenne du mélange constitué des donneurs de Michael (a) et des accepteurs de Michael (b)étant supérieure à 2.

**2.** Utilisation selon la revendication 1, caractérisée en ce que la composition contient une base servant de catalyseur (c).

**3.** Utilisation selon la revendication 1 ou 2, caractérisée en ce que $n + m$ est $\neq 0$.

**4.** Utilisation selon l'une des revendications 1 à 3, caractérisée en ce que la composition est durcie en l'absence d'un solvant.

**5.** Utilisation selon l'une des revendications 1 à 4, caractérisée en ce que, comme donneur de Michael (a), on utilise des acétoacétates ayant trois ou quatre groupes acétoacétoxy, dans lesquels $R^2$ est un atome d'hydrogène.

**6.** Utilisation selon l'une des revendications 1 à 5, caractérisée en ce que, comme accepteur de Michael (b), on utilise des esters d'un acide acrylique ayant trois ou quatre groupes acrylate, dans lesquels $R^5$ est un atome d'hydrogène.

**7.** Utilisation selon l'une des revendications 2 à 6, caractérisée en ce que, comme base servant de catalyseur (c), on utilise un alcoxyde d'un métal alcalin, un hydroxyde d'un tétra-alkylammonium, de l'amidine bicyclique et de la guanidine.

**8.** Utilisation selon l'une des revendications 1 à 7, caractérisée en ce qu'on utilise un excès stoechiométrique de l'accepteur de Michael (b) par rapport au donneur de Michael (a).

**9.** Utilisation selon l'une des revendications 1 à 8, caractérisée en ce que la composition contient un agent modificateur (d) qui présente au moins un groupe polymérisable de façon radicalaire, dont la réactivité vis à vis du donneur de Michael (a) est plus faible que celle des groupes acrylate $R^4$ de l'accepteur de Michael (b).

**10.** Utilisation selon l'une des revendications 1 à 9, caractérisée en ce que la composition est utilisée comme matière de remplissage d'un matériau dentaire.

**11.** Utilisation selon l'une des revendications 1 à 9, caractérisée en ce que le matériau dentaire est un composite de remplissage, une colle dentaire, un ciment de fixation ou un matériau de moulage.

**12.** Utilisation selon l'une des revendications 1 à 11, caractérisée en ce que la composition est utilisée sous la forme durcie.

**13.** Utilisation selon la revendication 12, caractérisée en ce que le matériau dentaire est une dent artificielle, un inlay, un onlay ou une couronne.

**14.** Corps dentaire façonné, pour le remplacement ou la reconstruction de dents, caractérisé en ce que le corps dentaire est formé à partir d'une composition durcie telle qu'utilisée selon les revendications 1 à 9.

**15.** Corps dentaire selon la revendication 14, caractérisé en ce que le corps dentaire se présente sous la forme d'une dent artificielle, d'un inlay, d'un onlay, d'une couronne, d'une prothèse ou d'une partie d'une prothèse.